(19) Europäisches Patentamt / European Patent Office / Office européen des brevets

(11) **EP 3 241 515 B1**

(12) **EUROPEAN PATENT SPECIFICATION**

(45) Date of publication and mention
of the grant of the patent:
**25.12.2019 Bulletin 2019/52**

(51) Int Cl.:
***A61B 18/14*** *(2006.01)*    *A61B 18/00* *(2006.01)*

(21) Application number: **17166390.9**

(22) Date of filing: **12.04.2017**

(54) **ABLATION MEDICAL DEVICE**

MEDIZINISCHE ABLATIONSVORRICHTUNG

DISPOSITIF MÉDICAL D'ABLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.04.2016 US 201662322866 P**

(43) Date of publication of application:
**08.11.2017 Bulletin 2017/45**

(73) Proprietor: **Cook Medical Technologies LLC
Bloomington, IN 47404 (US)**

(72) Inventors:
• **ELGAARD, Per
4690 Haslev (DK)**

• **PAAMAND, Rune Tore
2700 Broenshoej (DK)**
• **TORP, Allan
4632 Bjaeverskov (DK)**
• **BUTZBACKER, Raimo Urban
4690 Haslev (DK)**

(74) Representative: **Garratt, Peter Douglas et al
Mathys & Squire LLP
The Shard
32 London Bridge Street
London SE1 9SG (GB)**

(56) References cited:
**US-A- 6 165 172        US-A1- 2005 234 439
US-A1- 2014 276 742     US-B1- 6 582 423**

**Description**

FIELD

The present disclosure relates generally to medical devices. More specifically, the disclosure relates to a device and method(s) for occluding or closing a body vessel using a radiofrequency signal to transmit energy, heat, and/or ablate the body vessel.

BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.

It is also known to seek to constrict a vessel by endoluminal ablation, causing contraction of the vessel and/or coagulation of blood to form a blood clot in the vessel. Various methods can be employed to cause such ablation.

Reference is directed to US 6582423 which discloses electrosurgical apparatus for maintaining patency in virtually any hollow body passage which may be subject to occlusion by invasive cellular growth or invasive solid tumor growth. The apparatus is particularly useful for reducing or eliminating the effects of restenosis in coronary arteries by selectively removing tissue ingrowth in or around stents anchored therein. A tissue ablating region of a catheter progresses through atheromatous media or thrombus within a body lumen. A particular advantage is the confinement of current flow paths between a return electrode (hollow guide wire) and one or more active electrodes to the vicinity of the tissue ablating region.

Reference is further directed to US 6165172 which discloses a catheter including a plurality of primary leads to deliver energy for ligating a hollow anatomical structure. Each of the primary leads includes an electrode located at the working end of the catheter. Separation is maintained between the primary leads such that each primary lead can individually receive power of selected polarity. The primary leads are constructed to expand outwardly to place the electrodes into apposition with a hollow anatomical structure. High frequency energy can be applied from the leads to create a heating effect in the surrounding tissue of the anatomical structure. The diameter of the hollow anatomical structure is reduced by the heating effect, and the electrodes of the primary leads are moved closer to one another. Where the hollow anatomical structure is a vein, energy is applied until the diameter of the vein is reduced to the point where the vein is occluded. A balloon is inflated to occlude the structure before the application of energy. Where the structure is a vein, the inflated balloon obstructs blood flow and facilitates the infusion of saline, medication, or a high-impedance fluid to the vein in order to reduce the occurrence of coagulation and to improve the heating of the vein by the catheter. The catheter can include a lumen to accommodate a guide wire or to allow fluid delivery.

Reference is further directed to US 2014/0276742 which discloses a renal denervation system including an ablation catheter and an inflation balloon. The renal denervation catheter is insertable into a renal artery to perform a renal denervation procedure. The inflation balloon is inflatable within the renal artery, wherein one of a blood pressure condition in the renal artery resulting from operation of the inflation balloon and a performance characteristic of the inflation balloon indicates efficacy of the renal denervation procedure.

BRIEF SUMMARY

The disclosure may include any of the following aspects in various combinations and may also include any other aspect described below in the written description or in the attached drawings. This disclosure provides a medical device and methods for conducting vessel ablation and occlusion.

The device has two conductive elements or electrodes to generate or create an electrical field. The electrical field may be creating using a radiofrequency ("RF") signal or energy. One of skill in this art will appreciate that other types of energy sources can be used to create the electrical field. The electrical field generated by the device transmits energy to the body vessel having a blood flow.

The device includes a first elongate member having a proximal end and extending to a distal end, defining a longitudinal axis A. The distal end is atraumatic and includes a first conductive element. The device also includes a second elongate member having a first end adjacent the proximal end and extending to a second end proximal the distal end. The second end is also atraumatic and includes a second conductive element such that the second conductive element is longitudinally offset and radially spaced from the first conductive element. This longitudinally offset and radially spaced geometry creates a space or separation volume within the body vessel through which the energy generated by the device is transmitted to the surrounding blood, vessel wall, and other body vessel anatomy to cause ablation.

The device also includes a control unit operatively coupled to the first and second conductive elements. The control unit and the first and second conductive elements form an electrical circuit such that the control unit actuates the device to transmit energy through the separate volume and to the body vessel to cause ablation and/or occlude the vessel.

In another aspect, the device is part of an assembly such that the assembly transmits energy (e.g. radiofrequency energy or signal) to the body vessel and blood flow. The device includes any of the aspects discussed above, in addition to an outer sheath. The outer sheath has a first sheath end and extending to a second sheath end and forms a lumen therethrough. The first elongate member and/or second elongate member is slidably received within the lumen to facilitate delivery of the device to the target site for ablation.

Additionally in another aspect, the device is used in a method of treatment. The method includes positioning the medical device having any of the aspects described above in the body vessel at a target site and, subsequently, operating the device to transmit energy to the body vessel.

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

Various additional features and embodiments will become apparent with the following description. The present disclosure may be better understood by referencing the accompanying figures.

BRIEF DESCRIPTION OF THE DRAWINGS

Fig. 1 depicts a partial, environmental view of a medical device in accordance with one embodiment of the present disclosure;
Fig. 2 depicts a side view of the device of Fig. 1;
Fig. 3A depicts a partial, retracted view of the device of Fig. 1;
Fig. 3B depicts a partial, extended view of the device of Fig. 1; and
Fig. 4 depicts steps of a method of use of the device of Fig. 1 in accordance with one embodiment of the present disclosure.

DETAILED DESCRIPTION

The present disclosure will now be described more fully with reference to the accompanying figures, which show various embodiments. The accompanying figures are provided for general understanding of various embodiments and method steps. However, this disclosure may be embodied in many different forms. These figures should not be construed as limiting, and they are not necessarily to scale.

The following definitions will be used in this application.

"About" or "substantially" mean that a given quantity (e.g. lengths or volumes) is within 10%, preferably within 5%, more preferably within 1% of a stated value. For example, a first quantity of length can be within 10% of a second length quantity.

"Adjacent" referred to herein is near, near to, or in close proximity with.

"Longitudinally" and derivatives thereof will be understood to mean along the longitudinal axis of the device. "Longitudinally offset" will be understood to mean spaced along the longitudinal axis.

The terms "proximal" and "distal" and derivatives thereof will be understood in the frame of reference of a physician using the device. Thus, proximal refers to locations closer to the physician and distal refers to the locations farther away from the physician (e.g., deeper in the patient's vasculature).

"Radially" and derivatives thereof will be understood to mean along a radial axis of the body vessel.

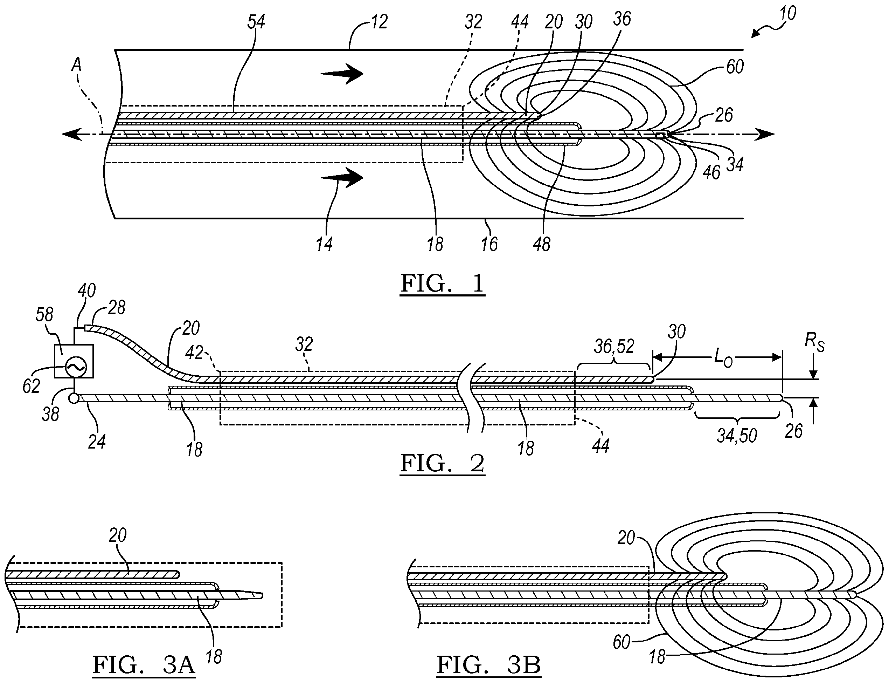

Fig. 1 depicts an environmental view of one aspect of the medical device 10 that may be used to apply energy to body vessel 12. As depicted, the body vessel 12 has a vessel wall 16 and a blood flow 14. A physician or other practitioner may desire to occlude or ablate the body vessel 12 at a target location. This involves using the device 10 to transmit energy to the body vessel 12 and the surrounding blood flow 14 (e.g. a local environment of the vessel).

The device 10 has a first elongate member 18 having a proximal end (Fig. 2 (24)) and extending to a distal end 26. The first elongate member 18 defines a longitudinal axis A. The distal end 26 is atraumatic so that it does not damage the vessel wall during tracking or use of the device 10. The distal end 26 includes a first conductive element 34.

Additionally, it will be understood to one skilled in the art that the first elongate member 18 being thin and/or the distal end 26 being atraumatic may have the advantage of allowing a practitioner to use this device in a small vessel. Therefore, it can be disadvantageous and/or undesirable to add additional features to the distal end 26 which increase the size of the distal end 26 relative to the body vessel 12 (e.g. radially extending wires), which may limit the use of this device 10 within a small vessel. The same is true for other parts of the device (e.g. the second elongate member 20).

The device has a second elongate member 20 having a first end (Fig. 2 (28)) positioned or disposed adjacent the proximal end (Fig. 2 (24)). The second elongate member 20 extends from the first end to a second end 30 also being atraumatic and proximal the distal end 26. The second end 30 includes a second conductive element 36. The second conductive element 36 is longitudinally offset and radially spaced from the first conductive element 34. In this arrangement, the first conductive element 34 is arranged generally in the center of the body vessel 12, while the second conductive element 36 is closer to the vessel wall 16.

The arrangement and geometry of the first and second conductive elements relative to each other creates a side-by-side, parallel relationship. As can be seen, the first elongate member is disposed next to and neither coaxial nor concentric with the second elongate member. This relationship and geometry could allow independent, radial movement of the two conductive elements relative to each other, giving the advantage of changing the location of the field created between them.

Independent movement of each electrode may enable the user to change the total area of electrode and to change the ratio of the electrode areas. The overall area may be changed to accommodate various output power levels, whereas the ratio will change the location of the field created and hence the heating pattern.

As discussed above, it will further be appreciated that the second elongate member can have similar characteristics to the first elongate member, being thin, atraumatic, and small to easily fit within the vessel. Either of the elongate members can be a modified wire guide including electrically conductive material and connected to a control unit and a power supply/signal generator.

Additionally or alternatively, either of the elongate members may form a loop or curved hook at their respective distal ends so that they come into closer contact with the vessel wall during use. These hooks could be small so as to keep the overall profile of the elongate member(s) thin, for the advantages discussed above. As the vessel wall swells, these curved distal ends may come into contact with the vessel wall and facilitate good heat transfer and faster ablation. For example, the first elongate member 18 can have a loop or hook at the distal end 26. Additionally or alternatively, either of the elongate members may have tapered tips at their respective distal ends to facilitate delivery of the device to the target site (e.g. Fig. 3A (18)).

The two conductive elements (34, 36) are operable to create an electrical field 60, generally shown in Fig. 1. The field 60 may be generated by the radiofrequency signal, and can heat the local environment, including the blood flow 14 and the vessel wall 16, to cause swelling and occluding of the body vessel 12 at a target site.

Generally using radiofrequency ("RF") signal, electrical terminal(s)/conductive element(s) are fed endoluminally into the vessel and an electrical pulse and/or constant electrical signal at RF frequencies is applied to the conductive element(s). The conductivity of blood and/or the vessel tissues causes localized heating of the blood and tissue and not significant heating of the conductive elements themselves, creating a local zone that is heated by the RF frequencies. This heating can be used to cause damage to the tissue (intima) of the vessel wall, resulting in vessel contraction. RF ablation heats the surrounding blood, causing this to coagulate around the electrical terminal and form a blood clot which blocks the vessel.

Two types of RF ablation apparatus are generally contemplated in the art: a monopolar system and a bipolar system. A monopolar system may have an elongate first electrode (e.g. active electrode) and a second electrode (e.g. dispersive electrode) pad or return electrode positioned outside the patient's body. The first electrode is designed to be fed endoluminally into the patient's vessel, while the second electrode pad is positioned against the person's outer body, as close as practicable to the first electrode. In a bipolar system, both electrodes are present in one device and inserted endoluminally into the vessel. Electrical energy applied to the first electrode will pass by conduction to the second electrode. There can be localized heating at the first and second electrodes, which effects the desired ablation.

Advantageously, operating the device having a monopolar system may be easier for manufacturing and use with multiple and/or different return electrode configurations. Alternatively advantageously, operating the device having a bipolar system may be easier for the user by not having a separable return electrode disposable outside of the body.

The RF frequencies, and the power they generate, may not be large enough to heat the first electrode and/or the second or return electrode. However, the RF signal and field generated will cause localized heating of the surrounding blood and tissue. The temperature of the electrodes remains at a first temperature (e.g. body temperature). Alternatively, the temperature of the electrodes can rise above body temperature.

In Fig. 1, the device 10 uses a bipolar system with the two conductive elements (34, 36) disposed in the vessel. Advantageously, operating the device having a bipolar system may be easier for the user by not having a separable return electrode disposable outside of the body.

The first and second conductive elements or electrodes (34, 36) are included in the first and second elongate members (18, 20), respectively, such that the first and second conductive elements (34, 36) are separated from each other by a fixed distance. However, one will appreciate that this distance could also be variable, as will be discussed further below.

Additionally, the device 10 can include a temperature sensor 46 (thermistor, thermocouple, and the like) integrated into the device at a suitable location. In Fig. 1, the temperature sensor 46 is located at or adjacent the distal end 26. In this aspect, the temperature sensor 46 could act as both a temperature sensor and the first conductive element/electrode 34. In a second aspect, the temperature sensor 46 could be electrically isolated from the first conductive element 34, but physically integrated with it (e.g. mounted on a first isolator 48) adjacent and/or proximal to the distal end 26.

In a third aspect, the temperature sensor could be integrated at one end (e.g. 44) of an outer sheath 32 (e.g. active or passive microcatheter). In a fourth aspect, the temperature sensor could be a separate device mounted at the tip of a delivery wire, allowing completely independent positioning of the temperature sensor relative to the conductive elements and other parts of the device.

In any case, sufficient electrical shielding may be needed between components of the device 10 conducting RF energy and the temperature signal because it can be difficult to sense temperature and conduct energy reliably if these components are too close to each other. One way of overcoming this interference is to alternate between ablation and temperature measurement so that ablation is performed out of phase with the temperature measurement. In one aspect, the practitioner may ablate for 900 milliseconds, and then measured temperature for 100 milliseconds, continuously repeating this 1 second pattern. Other time increments are also contemplated.

Another possible solution to the possible interference between temperature sensing and conducting energy is by electrical filtering of the temperature signal. Some examples include using RF chokes to block higher-frequency AC and/or low-pass filtering where the high-frequency interference is attenuated and the lower-frequency temperature signal is admitted.

It is also contemplated to use a first material for the temperature sensor 46 and a different, second material for the conductive elements (34, 36) to further avoid any interference. In one example, the temperature sensor and/or thermistor could be made of Nifethal® (a nickel/iron alloy), available from Kanthal, Hallstahmmar, Sweden. Temperature could be measured by the change in resistance of the material used.

Fig. 2 depicts further details of the device 10. Additionally, a control unit 58 is operatively coupled to the first and second conductive elements (34, 36), forming an electrical circuit such that the control unit 58 actuates the device to transmit energy to the body vessel. For example, the control unit includes a signal generator 62 capable of generating an RF or similar signal. The control unit 58 and the signal generator 62 may be directly or indirectly connected to the first and second conductive elements (34, 36). The spacing and medium (e.g. blood) between the first and second conductive elements closes the circuit or loop, forming an electrical circuit from these components.

In one aspect, the first elongate member 18 can be the first conductive element 34 (e.g. first electrode in AC system or anode in DC system) such that the first conductive element 34 extends from the proximal end 24 to the distal end 26. Additionally or alternatively, the second elongate member 20 can be the second conductive element 36 (e.g. second electrode in AC system or cathode in DC system) such that the second conductive element 36 extends from the first end 28 to the second end 30.

To electrically isolate the first and second conductive elements (34, 36), the first elongate member 18 may have a first isolator 48 disposed about its outer surface. The first isolator 48 may have a first isolator end distal the proximal end 24. The first isolator may extend to a second isolator end proximal the distal end 26, creating exposed ends of the first elongate member 18. In this way, even if the first conductive element 34 extends from the proximal end 24 to the distal end 26, it will have a central region electrically isolated from the second elongate member 20, the second conductive element 36, and the rest of the device.

The first isolator 48 includes a body disposed about the first elongate member 18 and having an outer surface. The second elongate member 20 can be immobilized on the outer surface of the first isolator 48. Alternatively, the second elongate member 20 could be slidable, not immobilized, adjacent to the first isolator 48. A lubricious coating on outer surface of the first isolator 48 could facilitate easy sliding.

The second elongate member 20 and the second conductive element 36 can be electrically isolated from the rest of the device by the first isolator 48 and a second isolator 54 disposed about the second conductive element 36. Either of the first and second isolators (48, 54) could be a shrink tubing heat shrunk and/or immoblized about or onto the elongate members, respectively.

The first and/or second isolators could include the Nifethal® alloy discussed herein. Each or both isolators (48, 56) could be thin and electrically insulating such that they provide suitable electrical insulation, but do not add significant thickness to prevent the device from being suitable for small vessels. Other materials and geometries for the isolators are possible. A lubricious coating could also be applied to any part of the device to facilitate delivery to the target site or slidably of the components relative to each other.

Alternatively, the second conductive element 36 could be at least partially electrically isolated from the rest of the device by a delivery and/or outer sheath 32. The outer sheath 32 is shown as a dotted line, and the internal components of the device are shown in solid lines. However, one of skill in this art will understand that the internal components would normally be obscured by the outer sheath 32. The outer sheath 32 may extend from a first sheath end 42 adjacent the proximal end 24 and to a second sheath end 44, forming a lumen therethrough. The first and second conductive elements (34, 36) would be slidably disposed in the lumen and movable relative to the outer sheath 32. In one aspect, the device could be provided with an outer sheath as an assembly. The assembly could include the outer sheath 32 having the first sheath end 42 and extending to the second sheath end 44 and forming a lumen therethrough. The first and second elongate members (18, 20) are slidably received within the lumen during delivery and use.

Fig. 2 also depicts details of the spacing or separation volume between the first and second conductive elements (34, 36). While the first conductive element 34 may be located at the distal end 26, it also may be located adjacent to the distal end 26, having a first length 50. Additionally or alternatively, the second conductive element 36 can be located at the second end 30, and/or it can be located adjacent to the second end 30 having a second length 52.

In one aspect, the first length 50 is shorter than the second length 52. Alternatively, the first length 50 may be approximately the same as the second length 52. The first length 50 and/or the second length 52 can each be about 5 millimeters to about 20 millimeters. In one example, the first length 50 and the second length 52 are each about 10 millimeters.

The first conductive element 34 is longitudinally offset by the second conductive element 36 by an offset length $L_o$. As discussed herein, the offset length $L_o$ can be fixed if the elements are immobilized relative to each other or variable if the elements are slidable relative to each other. Additionally, the first conductive element 34 is radially spaced along a radius of the device from the second conductive element 36 by a radial space $R_s$. This radial space can also be fixed or variable as discussed above.

The offset length $L_o$ and radial space $R_s$ create a separation space or separation volume, in the three dimensional environment of the device, where the first and second conductive elements (34, 36) are separated from each other. This separation volume will become filled by the blood flow and other components of the body vessel. These components will likely create the electrical resistance between the conductive elements (34, 36) and close the electrical circuit between the two to create the electrical field to transmit RF energy to the vessel wall, ablating the body vessel.

In one aspect, the RF energy could be an AC signal from about 100kHz to about 1MHz. More specifically, the AC signal could be about 400 to about 500kHz. The signal may also be a DC signal. The signal may vary over time, and can depend on the required power to maintain a desired temperature in the body vessel.

$$\text{Power} = \text{Current} \times \text{Voltage} \qquad \text{Voltage} = \text{Current} \times \text{Resistivity}$$

$$\text{Power} = \text{Current}^2 \times \text{Resistivity} = \text{Voltage}^2/\text{Resistivity}$$

Additionally, the electrical circuit can include other components or parts as needed and/or desired for form the circuit. For example, a first wire 40 can optionally be connected and form a junction between the control unit 58 and the second elongate member 20. A second wire 38 can optionally be connected to the control unit and form a junction between the control unit 58 and the first elongate member 18.

Figs. 3A-B depict different states of the device. For example, Fig. 3A depicts the elongate members (18, 20) being disposed or retracted into the outer sheath during delivery, in a delivery state. Fig. 3B depicts the elongate members being extended from the sheath for treatment, in a treatment state. In this example, the device is being operated to generate the electrical field 60. It will be understood by one skilled in the art that the electrical field 60 is merely illustrative of an electrical field between two electrodes, and the electrons within this field are not necessarily located in only the locations of these schematic lines.

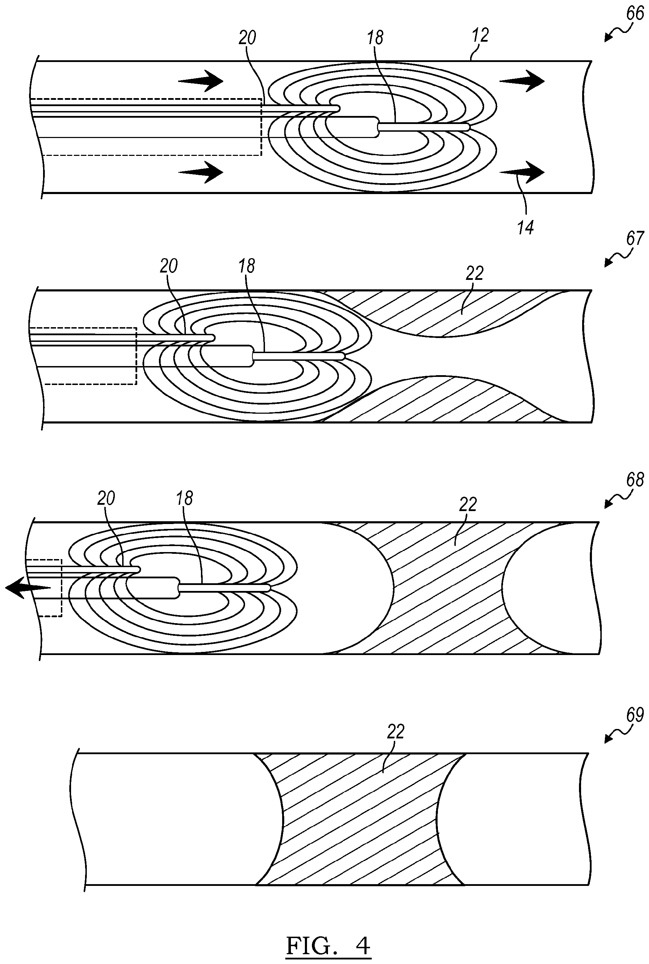

Fig. 4 depicts steps of one method to operate this device. In step 66, the device (including the elongate members (18, 20)) are delivered to the body vessel 12 at or adjacent the treatment site. In other words, the device is positioned in the body vessel. Blood flow 14 will flow around the device as the device is operated to create an electrical field. In step 67, the generated field will start to ablate the vessel wall, forming occlusion 22. The device can be optionally proximally retracted a short distance as the occlusion forms.

In step 68, the occlusion 22 can fully occlude the body vessel. In other words, the device is operated to transmit energy to the body vessel. In step 69, the device may be withdrawn from the treatment site such that the occlusion 22 remains intact. Through this method, the device ablates or occludes the body vessel, forming an occlusion to isolate diseased tissue, organs, tumors, and the like.

FEATURE COMBINATIONS

The following feature combinations assist in understanding this disclosure. The device includes a first elongate member having a proximal end and extending to a distal end, defining a longitudinal axis A. The distal end is atraumatic and includes a first conductive element. The device also includes a second elongate member having a first end adjacent the proximal end and extending to a second end proximal the distal end. The second end is also atraumatic and includes a second conductive element such that the second conductive element is longitudinally offset and radially spaced from the first conductive element. This longitudinal offset and radially spaced geometry creates a space or separation volume within the body vessel through which the energy generated by the device is transmitted to the surrounding blood, vessel wall, and other body vessel anatomy to cause ablation.

The device also includes a control unit operatively coupled to the first and second conductive elements. The control unit and the first and second conductive elements form an electrical circuit such that the control unit actuates the device to transmit energy through the separate volume and to the body vessel to cause ablation and/or occlude the vessel.

Additionally or alternatively to the above combination(s), the device can optionally include an outer sheath having a first sheath end adjacent the proximal end and a second sheath end adjacent the distal end, the outer sheath extending from the first sheath end to the second sheath end and forming a lumen therethrough, the first and second conductive elements being slidably disposed in the lumen.

Additionally or alternatively to the above combination(s), the device can optionally include an isolator disposed about the first elongate member and having a first isolator end distal the proximal end and extending to a second isolator end proximal the distal end, the first elongate member having a central region comprising the first conductive element such that the isolator electrically isolates the central region comprising the first conductive element from the second conductive element.

Additionally or alternatively to the above combination(s), the isolator can optionally include a body disposed about the first elongate member and has an outer surface, the second elongate member being immobilized on the outer surface.

Additionally or alternatively to the above combination(s), the isolator can optionally include a shrink tubing immobilized about the first elongate member.

Additionally or alternatively to the above combination(s), the distal end can optionally include the first elongate member being next to and neither concentric nor coaxial with the second elongate member.

Additionally or alternatively to the above combination(s), the first elongate member can optionally include a wire guide having a loop or hook at the distal end.

Additionally or alternatively to the above combination(s), the device can optionally include a temperature sensor.

Additionally or alternatively to the above combination(s), the first conductive element can optionally have a first length being about 5 millimeters to about 20 millimeters, and the second conducive element has a second length being about 5 millimeters to about 20 millimeters.

Additionally or alternatively to the above combination(s), the first conductive element can optionally have the first length is about 10 millimeters and the second length is about 10 millimeters.

Additionally or alternatively to the above combination(s), the first conductive element can optionally have the first length being shorter than the second length.

Additionally or alternatively to the above combination(s), the first conductive element can optionally be longitudinally offset from the second conducive element by an offset length being fixed or variable.

Additionally or alternatively to the above combination(s), the first conductive element can optionally be radially spaced from the second conducive element by a spacing length being about 1 millimeter to about 5 millimeters.

Additionally or alternatively to the above combination(s), the isolator is disposed between the first and second elongate members.

Additionally or alternatively to the above combination(s), the control unit can optionally include a signal generator operable to generate an alternating current.

Additionally or alternatively to the above combination(s), the device can optionally include an isolator disposed about the first elongate member and having a first isolator end adjacent the proximal end and extending to a second isolator end proximal the distal end, the first elongate member having a central region comprising the first conductive element such that the isolator electrically isolates the central region comprising the first conductive element from the second conductive element, the isolator being slidably received in the lumen.

Additionally or alternatively to the above combination(s), the device can optionally include a first connector and a second connector, the first connector extending from the control unit to the proximal end such that the first connector electrically couples the control unit and the first elongate member, the second connector extending from the control unit to the first end such that the second connector electrically couples the control unit and the second elongate member. Additionally or alternatively to the above combination(s), the first elongate member can optionally tapers at the distal end, and the second elongate member optionally tapers at the second end. There is herein disclosed a method to transmit energy to a body vessel having a blood flow, the method comprising:ositioning a medical device in the body vessel, the device comprising: an outer sheath having a first sheath end and extending to a second sheath end and forming a lumen therethrough; a first elongate member slidably received within the lumen and comprising a proximal end and extending to a distal end being atraumatic, defining a longitudinal axis, the elongate member comprising a first conductive element; a second elongate member comprising a first end adjacent the proximal end and extending to a second end being atraumatic and proximal the distal end, the second elongate member comprising a second conductive element such that the second conductive element is longitudinally offset and radially spaced from the first conductive element; and a control unit being operatively coupled to the first and second conductive elements, forming an electrical circuit such that the control unit actuates the device to transmit energy to the body vessel; and operating the device to transmit energy to the body vessel.

It should be understood that the foregoing relates to exemplary embodiments of the disclosure and that modifications may be made without departing from the scope of the disclosure as set forth in the following claims. While the disclosure has been described with respect to certain embodiments it will be appreciated that modifications and changes may be made by those skilled in the art without departing from the disclosure.

## Claims

1. A device to transmit energy to a body vessel having a blood flow, the device comprising:

   a first elongate member (18) comprising a proximal end and extending to a distal end, defining a longitudinal axis, the distal end being atraumatic and including a first conductive element (34);
   a second elongate member (20) comprising a first end adjacent the proximal end and extending to a second end proximal the distal end, the second end being atraumatic and including a second conductive element (36) such that the second conductive element is longitudinally offset by an offset length being fixed or variable and radially spaced from the first conductive element; and
   a control unit (58) being operatively coupled to the first and second conductive elements, forming an electrical circuit such that the control unit actuates the device to transmit energy to the body vessel;
   wherein the first elongate member has an isolator (48) disposed about its outer surface;
   wherein the isolator includes a body disposed about the first elongate member and having an outer surface; and
   wherein the second elongate member is immobilized on the outer surface of the isolator.

2. The device of claim 1 further comprising an outer sheath having a first sheath end adjacent the proximal end and a second sheath end adjacent the distal end, the outer sheath extending from the first sheath end to the second sheath end and forming a lumen therethrough, the first and second conductive elements being slidably disposed in the lumen.

3. The device of claim 1 or claim 2, the isolator having a first isolator end distal the proximal end and extending to a second isolator end proximal the distal end, the first elongate member having a central region comprising the first conductive element such that the isolator electrically isolates the central region comprising the first conductive element from the second conductive element.

4. The device of any one of the preceding claims wherein the first elongate member is next to and not concentric with the second elongate member.

5. The device of any one of the preceding claims wherein the first elongate member comprises a wire guide having a loop or hook at the distal end.

6. The device of any one of the preceding claims further comprising a temperature sensor (46).

7. The device of any one of the preceding claims wherein the first conducive element has a first length being about 5 millimeters to about 20 millimeters and preferably about 10 millimeters, and the second conducive element has a second length being about 5 millimeters to about 20 millimeters and preferably about 10 millimeters, preferably wherein the first length is shorter than the second length.

8. The device of any one of the preceding claims wherein the first conductive element is radially spaced from the second conducive element by a spacing length being about 1 millimeter to about 5 millimeters.

9. The device of claim 3 wherein the isolator is disposed between the first and second elongate members.

10. The device of any one of the preceding claims wherein the control unit comprises a signal generator operable to generate an alternating current.

11. The device of claim 2, the isolator disposed about the first elongate member and having a first isolator end adjacent the proximal end and extending to a second isolator end proximal the distal end, the first elongate member having a central region comprising the first conductive element such that the isolator electrically isolates the central region comprising the first conductive element from the second conductive element, the isolator being slidably received in the lumen.

12. The device of any one of the preceding claims further comprising a first connector and a second connector, the first connector extending from the control unit to the proximal end such that the first connector electrically couples the control unit and the first elongate member, the second connector extending from the control unit to the first end such that the second connector electrically couples the control unit and the second elongate member.

13. The device of any one of the preceding claims wherein the first elongate member tapers at the distal end, and the second elongate member tapers at the second end.

**Patentansprüche**

1. Vorrichtung zum Übertragen von Energie auf ein Körpergefäß mit einer Blutströmung, wobei die Vorrichtung das Folgende umfasst:

   ein erstes längliches Element (18), das ein proximales Ende umfasst und sich zu einem distalen Ende erstreckt, eine Längsachse definierend, wobei das distale Ende atraumatisch ist und ein erstes leitfähiges Element (34) aufweist;
   ein zweites längliches Element (20), das ein erstes Ende neben dem proximalen Ende umfasst und sich zu einem zweiten Ende proximal zum distalen Ende erstreckt, wobei das zweite Ende atraumatisch ist und ein zweites leitfähiges Element (36) aufweist, so dass das zweite leitfähige Element um eine Versatzlänge, die festgelegt oder variabel ist, längs versetzt und radial vom ersten leitfähigen Element beabstandet ist; und
   eine Steuereinheit (58), die funktionsfähig mit den ersten und zweiten leitfähigen Elementen gekoppelt ist, wodurch eine elektrische Schaltung gebildet wird, so dass die Steuereinheit die Vorrichtung zum Übertragen von Energie zum Körpergefäß betätigt;
   wobei das erste längliche Element einen Isolator (48) aufweist, der um seine Außenfläche angeordnet ist;
   wobei der Isolator einen Körper aufweist, der um das erste längliche Element angeordnet ist und eine Außenfläche aufweist; und
   wobei das zweite längliche Element auf der Außenfläche des Isolators immobilisiert ist.

2. Vorrichtung nach Anspruch 1, ferner umfassend eine Außenhülle mit einem ersten Hüllenende neben dem proximalen Ende und einem zweiten Hüllenende neben dem distalen Ende, wobei sich die Außenhülle vom ersten Hüllenende zum zweiten Hüllenende erstreckt und ein Lumen dort hindurch bildet, wobei die ersten und zweiten leitfähigen Elemente verschiebbar im Lumen angeordnet sind.

3. Vorrichtung nach Anspruch 1 oder Anspruch 2, wobei der Isolator ein erstes Isolatorende distal vom proximalen Ende aufweist und sich zu einem zweiten Isolatorende proximal vom distalen Ende erstreckt, wobei das erste längliche Element einen mittigen Bereich aufweist, der das erste leitfähige Element umfasst, so dass der Isolator den mittigen Bereich, der das erste leitfähige Element umfasst, vom zweiten leitfähigen Element elektrisch isoliert.

4. Vorrichtung nach einem der vorhergehenden Ansprüche, wobei das erste längliche Element neben dem zweiten länglichen Element liegt und nicht mit diesem konzentrisch ist.

5. Vorrichtung nach einem der vorhergehenden Ansprüche, wobei das erste längliche Element einen Führungsdraht mit einer Schlaufe oder einem Haken am distalen Ende umfasst.

6. Vorrichtung nach einem der vorhergehenden Ansprüche, ferner umfassend einen Temperatursensor (46).

7. Vorrichtung nach einem der vorhergehenden Ansprüche, wobei das erste leitfähige Element eine erste Länge von ungefähr 5 Millimeter bis ungefähr 20 Millimeter und vorzugsweise ungefähr 10 Millimeter aufweist und das zweite leitfähige Element eine zweite Länge von ungefähr 5 Millimeter bis ungefähr 20 Millimeter und vorzugsweise ungefähr 10 Millimeter aufweist, wobei die erste Länge vorzugsweise kürzer als die zweite Länge ist.

8. Vorrichtung nach einem der vorhergehenden Ansprüche, wobei das erste leitfähige Element um eine Abstandslänge von ungefähr 1 Millimeter bis ungefähr 5 Millimeter vom zweiten leitfähigen Element radial beabstandet ist.

9. Vorrichtung nach Anspruch 3, wobei der Isolator zwischen den ersten und zweiten länglichen Elementen angeordnet ist.

10. Vorrichtung nach einem der vorhergehenden Ansprüche, wobei die Steuereinheit einen Signalgenerator umfasst, der zum Erzeugen eines Wechselstroms betätigbar ist.

11. Vorrichtung nach Anspruch 2, wobei der Isolator um das erste längliche Element angeordnet ist und ein erstes Isolatorende neben dem proximalen Ende aufweist und sich zu einem zweiten Isolatorende proximal vom distalen Ende erstreckt, wobei das erste längliche Element einen mittigen Bereich aufweist, der das erste leitfähige Element umfasst, so dass der Isolator den mittigen Bereich, der das erste leitfähige Element umfasst, vom zweiten leitfähigen Element elektrisch isoliert, wobei der Isolator verschiebbar im Lumen aufgenommen ist.

12. Vorrichtung nach einem der vorhergehenden Ansprüche, ferner umfassend ein erstes Verbindungsglied und ein zweites Verbindungsglied, wobei sich das erste Verbindungsglied von der Steuereinheit zum proximalen Ende erstreckt, so dass das erste Verbindungsglied die Steuereinheit und das erste längliche Element elektrisch miteinander verbindet, wobei sich das zweite Verbindungsglied von der Steuereinheit zum ersten Ende erstreckt, so dass das zweite Verbindungsglied die Steuereinheit und das zweite längliche Element elektrisch miteinander verbindet.

13. Vorrichtung nach einem der vorhergehenden Ansprüche, wobei das erste längliche Element sich am distalen Ende verjüngt und das zweite längliche Element sich am zweiten Ende verjüngt.

## Revendications

1. Dispositif pour transmettre une énergie à un vaisseau sanguin ayant un écoulement de sang, le dispositif comprenant :

    un premier élément allongé (18) comprenant une extrémité proximale et s'étendant vers une extrémité distale, définissant un axe longitudinal, l'extrémité distale étant atraumatique et comprenant un premier élément conducteur (34) ;
    un second élément allongé (20) comprenant une première extrémité adjacente à l'extrémité proximale et s'étendant vers une seconde extrémité proximale à l'extrémité distale, la seconde extrémité étant atraumatique et comprenant un second élément conducteur (36) de sorte que le second élément conducteur soit décalé longitudinalement par une longueur de décalage fixe ou variable et espacé radialement du premier élément conducteur ; et
    une unité de commande (58) couplée fonctionnellement aux premier et second éléments conducteurs, formant un circuit électrique de sorte que l'unité de commande actionne le dispositif afin de transmettre une énergie au vaisseau corporel ;
    dans lequel le premier élément allongé possède un isolateur (48) disposé autour de sa surface externe ;
    dans lequel l'isolateur comprend un corps disposé autour du premier élément allongé et possédant une surface externe ; et
    dans lequel le second élément allongé est immobilisé sur la surface externe de l'isolateur.

2. Dispositif selon la revendication 1 comprenant en outre une gaine externe possédant une première extrémité de gaine adjacente à l'extrémité proximale et une seconde extrémité de gaine adjacente à l'extrémité distale, la gaine externe s'étendant depuis la première extrémité de gaine vers la seconde extrémité de gaine et formant une lumière à travers celle-ci, les premier et second éléments conducteurs étant disposés de manière coulissante dans la lumière.

3. Dispositif selon la revendication 1 ou la revendication 2, l'isolateur possédant une première extrémité d'isolateur distale à l'extrémité proximale et s'étendant vers une seconde extrémité d'isolateur proximale à l'extrémité distale, le premier élément allongé possédant une région centrale comprenant le premier élément conducteur de sorte que l'isolateur isole électriquement la région centrale comprenant le premier élément conducteur du second élément conducteur.

4. Dispositif selon l'une quelconque des revendications précédentes dans lequel le premier élément allongé est à côté du second élément allongé et non concentrique à celui-ci.

5. Dispositif selon l'une quelconque des revendications précédentes dans lequel le premier élément allongé comprend un guide-fil possédant une boucle ou un crochet au niveau de l'extrémité distale.

6. Dispositif selon l'une quelconque des revendications précédentes comprenant en outre un capteur de température (46).

7. Dispositif selon l'une quelconque des revendications précédentes dans lequel le premier élément conducteur a une première longueur d'environ 5 millimètres à environ 20 millimètres et de préférence d'environ 10 millimètres, et le second élément conducteur a une seconde longueur d'environ 5 millimètres à environ 20 millimètres et de préférence d'environ 10 millimètres, de préférence dans lequel la première longueur est plus courte que la seconde longueur.

8. Dispositif selon l'une quelconque des revendications précédentes dans lequel le premier élément conducteur est espacé radialement du second élément conducteur par une longueur d'espacement d'environ 1 millimètre à environ 5 millimètres.

9. Dispositif selon la revendication 3 dans lequel l'isolateur est disposé entre les premier et second éléments allongés.

10. Dispositif selon l'une quelconque des revendications précédentes dans lequel l'unité de commande comprend un générateur de signaux permettant de générer un courant alternatif.

11. Dispositif selon la revendication 2, l'isolateur étant disposé autour du premier élément allongé et possédant une première extrémité d'isolateur adjacente à l'extrémité proximale et s'étendant vers une seconde extrémité d'isolateur proximale à l'extrémité distale, le premier élément allongé possédant une région centrale comprenant le premier élément conducteur de sorte que l'isolateur isole électriquement la région centrale comprenant le premier élément conducteur du second élément conducteur, l'isolateur étant accueilli de manière coulissante dans la lumière.

12. Dispositif selon l'une quelconque des revendications précédentes comprenant en outre un premier connecteur et un second connecteur, le premier connecteur s'étendant depuis l'unité de commande vers l'extrémité proximale de sorte que le premier connecteur couple électriquement l'unité de commande et le premier élément allongé, le second connecteur s'étendant depuis l'unité de commande vers la première extrémité de sorte que le second connecteur couple électriquement l'unité de commande et le second élément allongé.

13. Dispositif selon l'une quelconque des revendications précédentes dans lequel le premier élément allongé diminue au niveau de l'extrémité distale, et le second élément allongé diminue au niveau de la seconde extrémité.

FIG. 1

FIG. 2

FIG. 3A

FIG. 3B

EP 3 241 515 B1

FIG. 4

**EP 3 241 515 B1**

**REFERENCES CITED IN THE DESCRIPTION**

**Patent documents cited in the description**

- US 6582423 B
- US 6165172 A
- US 20140276742 A